# FASCICULE DE BREVET EUROPEEN

(11) **EP 3 280 704 B1**
(45) Date de publication et mention de la délivrance du brevet: **12.06.2019**
(21) Numéro de dépôt: 16729302.6
(22) Date de dépôt: 08.04.2016
(51) Int. Cl.: C07D 303/40, C07C 67/31, C07C 69/732, C07D 307/33

(54) **PROCEDE DE SYNTHESE D'UN PRECURSEUR D'UN UNIQUE ISOMERE DE DAIRY-LACTONE**
VERFAHREN ZUR SYNTHESE EINES VORLÄUFERS EINES EINZIGEN MILCH-LACTON-ISOMERS
METHOD FOR SYNTHESIZING A PRECURSOR OF A SINGLE DAIRY-LACTONE ISOMER

(30) Priorité: 10.04.2015 FR 1553112
(43) Date de publication de la demande: 14.02.2018
(73) Titulaire: Institut des Sciences et Industries du Vivant et de l'Environnement - AgroParisTech, 75231 Paris Cedex 05 (FR); University of New England, New South Wales 2351 (AU); Circa Group Pty Ltd., Coburg North, Victoria 3058 (AU)
(72) Inventeur: ALLAIS, Florent, 51400 Bouy (FR); FLOURAT, Amandine, 51100 Reims (FR); PERU, Aurélien, 51100 Reims (FR); GREATREX, Ben, Black Mountain, New South Wales 2365 (AU); WARWICK, Douglas, Viewbank, Victoria 3084 (AU); DUNCAN, Anthony, Brighton, Victoria 3186 (AU)
(74) Mandataire: IP Trust
(86) Numéro de dépôt international: PCT/FR2016/050813
(87) Numéro de publication internationale: WO 2016/162646

(56) Documents cités:
- EP-A2- 0 578 388
- ANDREAS HABEL ET AL: "Efficient and flexible synthesis of chiral [gamma]- and [delta]-lactones", ORGANIC & BIOMOLECULAR CHEMISTRY, vol. 6, no. 9, 2008, page 1601, XP055215231, ISSN: 1477-0520, DOI: 10.1039/b801514g & Andreas Habel ET AL: "Efficient and flexible Synthesis of Chiral [gamma]-and [delta]-Lactones Supporting Information", Efficient and flexible synthesis of chiral [gamma]- and [delta]-lactones (Supporting Information), 10 mars 2008 (2008-03-10), XP055215230, Extrait de l'Internet: URL:http://www.rsc.org/suppdata/ob/b8/b801 514g/b801514g.pdf [extrait le 2015-09-22]
- M.MARK MIDLAND ET AL: "The synthesis of naturally occurring 4-alkyl- and 4-alkenyl-[gamma]-lactones using the asymmetric reducing agent -3-pinanyl-9-borabicyclo[3.3.1]nonane", TETRAHEDRON LETTERS, vol. 21, no. 37, 1980, pages 3549-3552, XP055214838, ISSN: 0040-4039, DOI: 10.1016/0040-4039(80)80232-2
- P. HAVERKAMP BEGEMANN ET AL: "The synthesis of lactones. Part III: [delta]-Enollactones and lactones with exocyclic double bonds", RECUEIL DES TRAVAUX CHIMIQUES DES PAYS-BAS, vol. 86, no. 12, 2 septembre 1967 (1967-09-02), pages 1335-1344, XP055215090, ISSN: 0165-0513, DOI: 10.1002/recl.19670861210
- E. I. HEIBA ET AL: "Oxidation by metal salts. X. One-step synthesis of .gamma.-lactones from olefins", JOURNAL OF THE AMERICAN CHEMICAL SOCIETY, vol. 96, no. 26, 1974, pages 7977-7981, XP055214946, ISSN: 0002-7863, DOI: 10.1021/ja00833a024
- STUART J G ET AL: "COBALT-MEDIATED ALKYLATION OF SILOXY FURANS", HETEROCYCLES : AN INTERNATIONAL JOURNAL FOR REVIEWS AND COMMUNICATIONS IN HETEROCYCLIC CHEMISTRY, JAPAN INSTITUTE OF HETEROCYCLIC CHEMISTRY, JP, vol. 32, no. 5, 1991, pages 949-963, XP001080667, ISSN: 0385-5414
- ERIC FREROT ET AL: "Easy Access to Aroma Active Unsaturated [gamma]-Lactones by Addition of Modified Titanium Homoenolate to Aldehydes", JOURNAL OF AGRICULTURAL AND FOOD CHEMISTRY, vol. 59, no. 8, 27 avril 2011 (2011-04-27) , pages 4057-4061, XP055214863, ISSN: 0021-8561, DOI: 10.1021/jf104711x
- TIMOTHY M. TRYGSTAD ET AL: "Versatile Synthesis of the C3-C14 Domain of 7-Deoxyokadaic Acid", THE JOURNAL OF ORGANIC CHEMISTRY, vol. 74, no. 2, 16 janvier 2009 (2009-01-16), pages 910-913, XP055215356, ISSN: 0022-3263, DOI: 10.1021/jo8017457

## Description

### Domaine de l'invention

La présente invention s'inscrit dans le domaine des molécules aromatiques, leur procédé de fabrication et leur utilisation.

Plus particulièrement, la présente invention porte sur un procédé de synthèse de dairy-lactone et différents précurseurs pour leur utilisation pour leurs propriétés organoleptiques dans l'agroalimentaire, la pharmacie, la cosmétique ou la parfumerie.

### Etat de la technique

L'on connaît de l'état de la technique un procédé de synthèse de la dairy-lactone par fermentation.

Le brevet EP0578388 décrit un procédé de fermentation pour préparer des dérivés d'acide 10-hydroxy-C18-carboxylic et les compositions les comprenant et des compositions comportant des dérivés de gamma-dodecalactone (dont la dairy-lactone) pour leurs utilisations pour leurs propriétés organoleptiques notamment pour augmenter ou corriger des parfums ou des arômes de parfums, articles parfumés, de produits alimentaires, chewing-gums, de dentifrice, produits capillaires, tabacs. Les gamma-lactones sont utiles en tant qu'arômes alimentaires (Eric Frerot et al, « Easy access to aroma active unsaturated (gamma)-lactones by addition of modified titanium homoenolate to aldehydes », Journal of agricultural and food chemistry, 2011).

L'on connaît également de l'état de la technique la dairy-lactone et ses énantiomères (Andreas Habel et al, « Efficient and flexible synthesis of chiral (gamma)- and (delta)--lactones » (Organic & biomolecular chemistry, 2008 ; M.Mark Midland et al, « the synthesis of naturally occuring 4-alkyl- and 4-alkenyl-(gamma)-lactones using asymetric reducing agent -3-pinanyl-9-borabicyclo(3.3.1)nonane », Tetrahedron letters, 1980).

Enfin l'on connaît également de l'état de la technique des procédés de synthèse de différents intermédiaires pouvant intervenir dans la synthèse de lactones (P.Haverkamp Begemann et al, « the synthesis of lactones. Part III : (delta)-enollactones and lactones with exocyclic double bonds », Recueil des travaux chimiques des Pays-Bas, 1967 ; E.I. Heiba et al, « oxydation by métal salts. X. One step synthesis of (gamma)-lactones from olefins », Journal of the American chemical society, 1974 ; Stuart JG et al, « Cobalt-mediated alkylation of siloxy furans », Heterocycles : an international journal for reviews and communications in heterocyclic chemistry, Japan institue of heterocyclic chemistry, 1991). La préparation d'un composé racémique de formule (VII) comme défini dans les revendications est divulguée dans le Recueil Des Travaux Chimiques des Pays Bas 1968, 87, 1335-1344.

### Inconvénients de l'art antérieur

Le procédé du brevet EP0578388 permet d'obtenir par fermentation un mélange racémique de dairy-lactone. L'obtention de dairy-lactone par fermentation nécessite l'utilisation successive de plusieurs espèces fermentaires ce qui représente un frein particulièrement important à l'industrialisation du procédé.

La fermentation entraîne l'obtention d'un mélange racémique de dairy-lactone. Or il est bien connu que deux énantiomères d'une même formule chimique peuvent avoir des propriétés radicalement différentes.

### Solution apportée par l'invention

Afin de remédier à ces inconvénients, la présente invention propose dans son acceptation la plus générale un procédé de préparation d'un précurseur d'un unique isomère de dairy-lactone comportant une étape de préparation d'un glycidyl-ester suivi d'une étape d'ouverture d'époxyde.

Le glycidyl-ester a la formule (V) suivante, où R₁ est un groupement alkyle, notamment un groupement CH₃ ou C₂H₅ et où la ligne en pointillée représente une liaison carbone-carbone simple ou double (de configuration Z ou E):

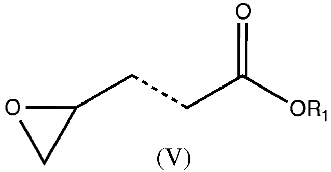

Le glycidyl-ester est préparé à partir d'une lactone de formule (III) ci-dessous uniquement de conformation R ou S, où la ligne en pointillée représente une liaison carbone-carbone simple ou double, de sorte que ledit glycidyl-ester et ledit précurseur soient sous forme isomérique pure uniquement de conformation R ou S, et que la dairy-lactone soit un isomère pur uniquement de forme R ou S correspondante.

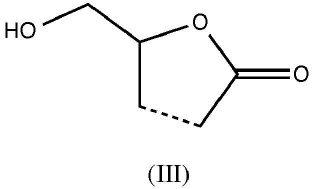

Contrairement au procédé de l'art antérieur, le procédé de la présente invention permet d'obtenir des énantiomères purs de chacun des précurseurs de dairy-lactone et également un énantiomère pur de dairy-lactone. Le procédé selon l'invention permet préférentiellement de synthétiser la (Z)-dairy-lactone, de formule C₁₂H₂₀0₂ et de formule développée (VIII) ci-dessous.

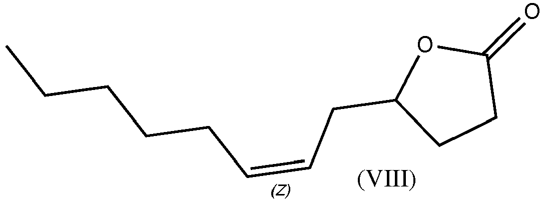

Le procédé selon l'invention permet d'obtenir rapidement et à moindre coût une solution pure de dairy-lactone. Préférentiellement une solution pure de Z-dairy-lactone est obtenue par le procédé conformément à l'invention.

L'étape de synthèse d'un glycidyl-ester consiste à l'ouverture de la lactone et l'élimination concertée d'un alcool activé d'une molécule de formule (IV) ci-dessous, où R₂ est choisi parmi les groupements tosyl (Ts) ou mésyl (Ms) et où la ligne en pointillée représente une liaison carbone-carbone simple ou double.

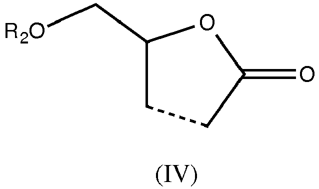

Cette molécule de formule (IV) est par exemple la (S)-(5-oxotetrahydrofuran-2-yl)methyl-4-methylbenzenesulfonate dans le cas où R₂ est un groupement tosyl et où la liaison carbone-carbone représentée par la ligne en pointillée est une liaison simple ; ou le (*S*)-(5-oxotetrahydrofuran-2-yl)-methyl methanesulfonate dans le cas où R₂ est un groupement mesyl et où la liaison carbone-carbone représentée par la ligne en pointillée est une liaison simple.

La molécule de formule (IV) est obtenue par activation de l'alcool par sulfonation d'une molécule de formule (III) suivante où la ligne en pointillée représente une liaison carbone-carbone simple ou double:

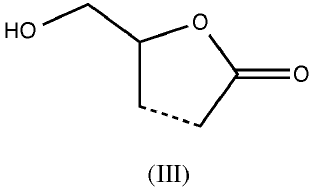

La sulfonation permet d'activer l'alcool afin de pouvoir le substituer par la suite. Cette sulfonation se fait par tosylation, par mésylation.

Une autre alternative pour l'étape de synthèse d'un glycidyl-ester consiste à l'ouverture de la lactone et l'élimination concertée d'un halogénure d'une molécule de formule (IV-Bis) ci-dessous, où X est choisi parmi Br, Cl ou I et où la ligne en pointillée représente une liaison carbone-carbone simple ou double.

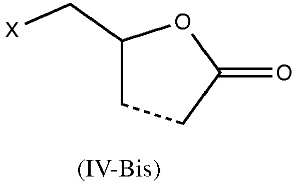

La molécule de formule (IV-bis) est obtenue par la substitution par un halogène de l'alcool d'une molécule de formule (III) où la ligne en pointillée représente une liaison carbone-carbone simple ou double.

Dans un mode de réalisation, la molécule de formule (IV) est obtenue par activation de l'alcool par sulfonation d'une lactone.

Alternativement, ladite molécule de formule (III) dans laquelle la ligne pointillée représente une liaison carbone-carbone simple est obtenue par hydrogénation d'une lactone α,β-insaturée.

La lactone α,β-insaturée est préférentiellement une lactone de formule (II) suivante :

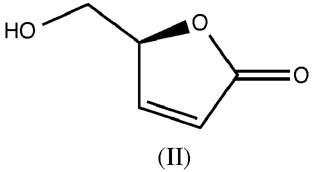

Cette lactone est préférentiellement le (S)-furanone (HFO) obtenue par modification de la lévoglucogénone provenant de la transformation de la cellulose conformément au procédé décrit dans la demande de brevet FR1453957.

Alternativement, la molécule de formule (III) dans laquelle la ligne pointillée représente une liaison carbone-carbone simple est obtenue par oxydation de la dihydrolévoglucosénone en présence d'un péracide, par exemple l'acide péracétique ou le MCPBA, suivie d'une hydrolyse acide ou basique du mélange de produits obtenu, par exemple en présence d'une résine acide. Cette dihydrolévoglucosénone est obtenue par hydrogénation d'une lévoglucosénone.

Avantageusement, le procédé de préparation d'un précurseur d'au moins un isomère de dairy-lactone comporte en outre une étape de lactonisation d'une molécule de formule (VI) ci-dessous, où R₃ est préférentiellement un groupement alkyle, par exemple un groupement CH₃ ou C₂H₅, et où la ligne en pointillée représente une liaison carbone-carbone simple ou double.

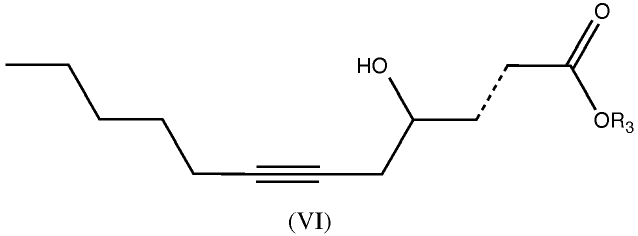

Cette molécule de formule (VI) est obtenue par ouverture d'époxyde d'un glycidyl-ester de formule (V) décrit précédemment.

La lactonisation de la molécule de formule (VI) est réalisable en milieu acide ou basique. Préférentiellement, elle est réalisée par hydrolyse acide. Cette lactonisation permet d'obtenir une molécule de formule (VII) où la ligne en pointillée représente une liaison carbone-carbone simple ou double.

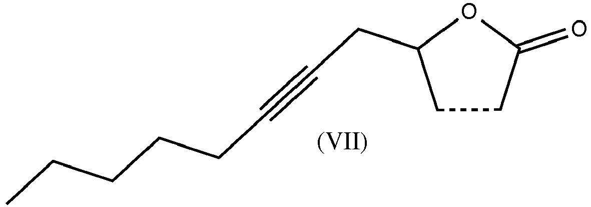

La molécule de formule (VII) où la ligne en pointillée représente une liaison carbone-carbone simple est par exemple le (*S*)-5-(oct-2yn-1-yl)-dihydrofuran-2(3*H*)-one. La molécule de formule (VII) où la ligne en pointillée représente une liaison carbone-carbone double est par exemple le (*R*)-5-(oct-2-yn-1-yl)-furan-2(5H)-one.

Le procédé de préparation d'un précurseur d'au moins un isomère de dairy-lactone comporte en outre une étape de syn-hydrogénation d'une molécule de formule (VII).

Cette syn-hydrogénation de la molécule de formule (VII) aboutit à la synthèse du produit final, la dairy-lactone ayant des propriétés organoleptiques particulièrement intéressantes. L'on comprend que l'ensemble des étapes susmentionnées aboutit à la synthèse d'un unique isomère de dairy-lactone.

Selon une alternative, la synthèse de dairy-lactone à partir de la molécule de la formule (VII) comportant une double liaison dans le cycle implique une syn-hydrogénation permettant, en une seule étape, la réduction de l'alcyne en alcène de configuration Z et celle de l'insaturation de la lactone α,β-insaturée.

Alternativement, l'énantiomère de forme E de dairy-lactone est obtenue par hydrogénation.

Selon un mode de réalisation, le procédé de préparation d'un unique isomère de dairy-lactone comporte les étapes précédemment énumérées.

La dairy-lactone obtenue est un énantiomère pur de toutes les conformations possibles. L'isomère de dairy-lactone obtenu par le procédé est pur et de forme Z-R, Z-S, E-R ou E-S. Préférentiellement, la dairy-lactone obtenue est la Z-dairy-lactone. Encore plus préférentiellement, la dairy-lactone obtenue par le procédé est la (Z,S)-dairy-lactone de formule (VIII) suivante :

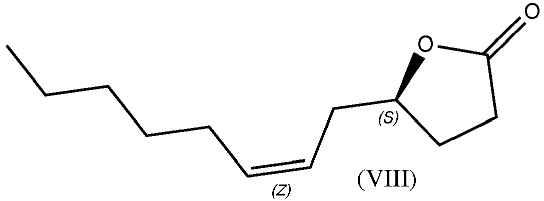

Cette (Z,S)-dairy-lactone de formule (VIII) possède des propriétés organoleptiques particulièrement intéressantes pour une utilisation comme parfum et/ou arôme en agro-alimentaire, en pharmacie, en cosmétique, en parfumerie...

Selon un aspect, l'invention porte sur un glycidyl-ester de formule (V), décrit ci-avant, où R₁ est un groupement alkyle pour synthétiser un isomère pur de précurseur de dairy-lactone et/ou un isomère pur de dairy-lactone.

L'utilisation d'un isomère pur de dairy-lactone comme arôme alimentaire est également décrite. L'utilisation d'un isomère pur de dairy-lactone obtenu par le procédé de la présente invention comme arôme alimentaire est également décrite.

L'utilisation d'un énantiomère pur de (Z,S)-dairy-lactone comme arôme alimentaire est également décrite.

L'utilisation d'un isomère pur de dairy-lactone comme molécule odorante pour parfumer un produit alimentaire est également décrite. L'utilisation d'un énantiomère pur de (Z,S)-dairy-lactone comme molécule odorante pour parfumer un produit alimentaire est également décrite. L'utilisation d'un isomère pur de dairy-lactone comme molécule odorante pour parfumer un produit cosmétique est également décrite. L'utilisation d'un énantiomère pur de (Z,S)-dairy-lactone comme molécule odorante pour parfumer un produit cosmétique est également décrite.

Un produit de boulangerie comportant un isomère pur de dairy-lactone pour conférer une note « beurrée » audit produit de boulangerie est également décrit. Selon un mode particulier de réalisation, le produit de boulangerie comporte un énantiomère pur de (Z,S)-dairy-lactone pour conférer une note « beurrée » audit produit de boulangerie.

Avantageusement, le produit de boulangerie contient 5 à 10 ppm de (Z,S)-dairy-lactone.

Un produit à base de lait végétal ou de substitut au lait animal comportant un isomère pur de dairy-lactone pour conférer une note « lactée » audit produit est également décrit. Selon un mode particulier de réalisation, le produit à base de lait végétal ou de substitut au lait animal comporte un isomère pur de (Z,S)-dairy-lactone pour conférer une note « lactée » audit produit.

### Description

La présente invention sera mieux comprise à la lumière d'un exemple non limitatif de réalisation.

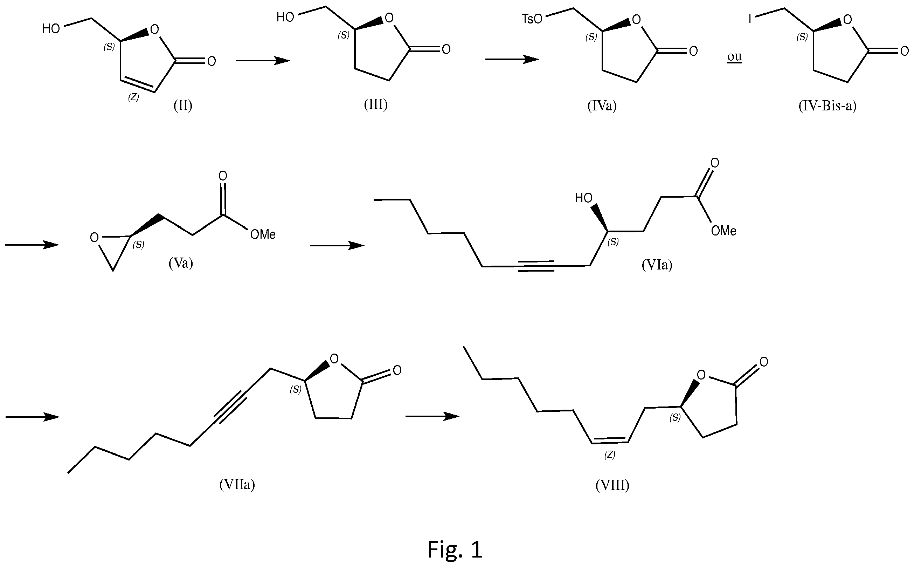

La figure 1 représente un schéma des différentes étapes du procédé de synthèse d'au moins un précurseur d'un isomère de la (Z,S)-dairy-lactone et d'un isomère de la (Z,S)-dairy-lactone à partir de la lactone (II).
La figure 2 représente un schéma de la synthèse enzymatique de précurseurs de la dairy-lactone, la (S)-furanone (molécule II) et de la 4-hydroxyméthylbutyrolactone (molécule III) et est décrit aux exemples 1 et 2.
La figure 3 représente une voie alternative de synthèse de la 4-hydroxyméthylbutyrolactone (molécule III) par synthèse chimique et est décrite à l'exemple 2bis.

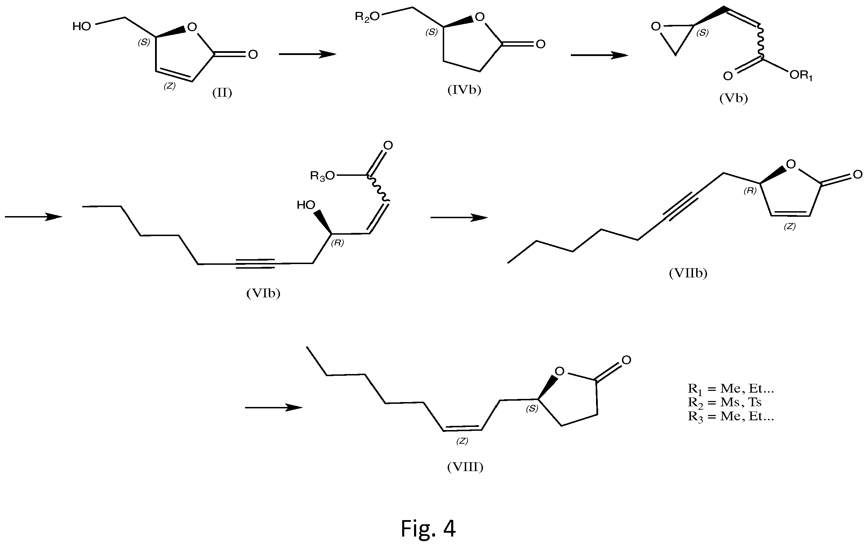

La figure 4 représente une voie alternative de synthèse d'au moins un précurseur d'un isomère de (Z,S)-dairy-lactone et d'un isomère de la (Z,S)-dairy-lactone.

### Exemple 1 : synthèse de (S)-furanone (HFO) de formule (II) à partir d'une cétone particulière, la lévoglucosénone

L'un quelconque des procédés décrits dans la demande de brevet FR1453957 peut être utilisé.

Le (S)-furanone ou (S)-4-hydroxyméthylbuténolide a la formule (II) suivante :

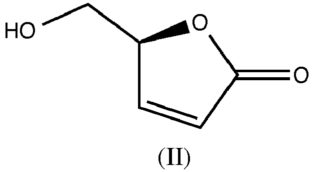

Du 4-hydroxyméthylbuténolide, de formule (II), est préparé à partir de lévoglucosénone (LGO), obtenue par valorisation de la biomasse, selon le mode de mise en oeuvre particulier du procédé dit en « one-pot ».

### Oxydation

Dans un réacteur, une solution aqueuse de peroxyde d'hydrogène H₂O₂ à 30% (2,57 mmol, 0,26 mL, 1,2 éq. par rapport à la LGO) est ajoutée en une unique portion à une suspension de LGO (270 mg, 2,14 mmol) et de lipase CaL-B (Novozym® 435, 75 mg, 315 U/mmol LGO) dans l'acétate d'éthyle (3 mL) sous agitation à température ambiante, dans un incubateur à agitation planaire. Pour cet exemple comme pour tous les exemples décrits ci-après, 1 g de Novozym® 435 correspond à 9000 unités de lipase CaL-B (activité mesurée après séjour de l'enzyme dans l'acétate d'éthyle). Le mélange réactionnel est agité à 40 °C pendant 4 h puis évaporé à sec.

### Hydrolyse acide

De l'acide chlorhydrique concentré (5 mmol, 0,4 mL) est ajouté à une solution de ce mélange brut dans le méthanol (5 mL), à température ambiante. Le mélange réactionnel est chauffé sous agitation pendant 8 à 16 heures, de sorte à convertir le formiate en l'alcool correspondant. Le mélange réactionnel est évaporé à sec avec un gel de silice. Le produit brut est purifié par chromatographie sur gel de silice (élution avec 75 à 100% d'acétate d'éthyle dans du cyclohexane) pour obtenir du (S)-4-hydroxyméthylbuténolide de formule (II) pur (175 mg, 72%).
¹H RMN (CDCl₃): d 7,53 (dd, *J* = 1,5 et 5,7 Hz, 1 H), 6,2 (dd, *J* = 1,5 et 5,7 Hz, 1 H), 5,17 (m, 1 H), 4,0 (d, *J* = 3,6 et 12,0 Hz, 1 H), 3,80 (dd, *J* = 3,6 et 12,0 Hz, 1 H)
¹³C RMN (CDCl₃): d 173,5 (s), 154,0 (d), 122,8 (d), 84,3 (d), 62,2 (t)

En variante, à l'issue de l'étape de traitement de la LGO avec la lipase, cette dernière est séparée du milieu réactionnel préalablement à l'étape d'évaporation à sec du milieu. L'hydrolyse acide est ensuite réalisée comme décrit ci-avant. On obtient également du 4-hydroxyméthylbuténolide pur, avec un même rendement de 72%.

Dans d'autres variantes du procédé, l'étape d'hydrolyse acide est réalisée directement sur le milieu réactionnel obtenu à l'issue de l'étape d'oxydation, sans qu'il soit réalisé au préalable d'étape d'évaporation à sec. Que la lipase ait été éliminée du milieu réactionnel par filtration ou non, dans de telles variantes, le rendement de la réaction est similaire à celui obtenu pour le mode de mise en oeuvre décrit ci-avant de manière détaillée, c'est-à-dire d'environ 72%.

### Exemple 2 : synthèse de 4-hydroxyméthylbutyrolactone de formule (III) à partir de (S)-furanone de formule (II)

Le (S)-4-hydroxyméthylbuténolide ou (S)-furanone de formule (II) obtenu à l'Exemple 1 est soumis à hydrogénation catalytique, de la manière suivante.

Du Pd/C (10 % p/p, 250 mg) est ajouté à une solution de 4-hydroxyméthylbuténolide (1,4 g, 12,3 mmol) dans de l'acétate d'éthyle (15 mL) à température ambiante. La suspension sous agitation est dégazée 3 fois sous vide/azote. La suspension est ensuite hydrogénée par une atmosphère d'hydrogène à température ambiante pendant 4 heures. Le mélange brut est filtré sur un tampon de célite et le filtrat est concentré à sec avec un gel de silice. Le produit brut est purifié par chromatographie sur gel de silice (élution avec un gradient allant de 75 à 100% d'acétate d'éthyle dans le cyclohexane), pour obtenir de la (S)-4-hydroxyméthylbutyrolactone de formule (III) pure (1,19 g, 82%).
¹H RMN (CDCl₃): d 4,64 (m, 1 H), 3,92 (dd, *J* = 2,7 et 12,6 Hz, 1 H), 3,66 (dd, *J* = 4,5 et 12,6 Hz, 1 H), 2,72-2,49 (m, 3 H), 2,35-2,09 (m, 2 H)
¹³C RMN (CDCl₃): d 177,7 (s), 80,8 (d), 64,1 (t), 28,7 (t), 23,1 (t)

Cette voie de synthèse permet d'obtenir, avec des rendements élevés la (S)-4-hydroxyméthylbutyrolactone de formule (III) et de forme S, dont la structure est confirmée par RMN du proton et du carbone, et par polarimétrie (pouvoir rotatoire).

### Exemple 2 bis : synthèse de (S)-4-hydroxyméthylbutyrolactone de formule (III) à partir de dihydrolévoglucosénone

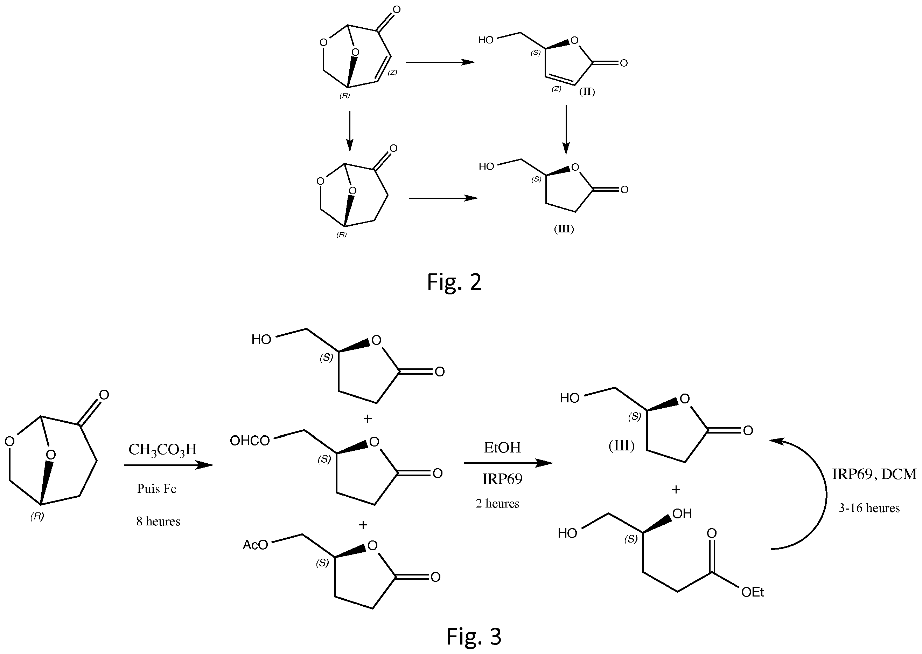

Cet exemple est une alternative à l'exemple 1 et 2 ci-dessus et est présenté en figure 3.

### (S) (+)-5-(Hydroxymethyl)dihydrofuran-2(3H)-one (III).

A une solution de dihydrolévoglucosénone (5.0 g, 39 mmol) dans l'eau (30 mL) est ajouté de l'acide péracétique à 32% (13 g, 55 mmol) sur une période de 30 minutes tout en maintenant la température du mélange entre 25 et 35 °C. Le mélange est agité à cette température pendant 8 heures puis est neutralisé en le versant sur de la limaille de fer (1.0 g) et en l'agitant jusqu'à disparition des péroxydes (test papier d'iode, 5 minutes) puis pour une période supplémentaire de 16 heures. Le mélange est ensuite filtré sur Célite et concentré sous pression réduite pour donner un mélange huile-solide. Ce dernier est ensuite suspendu dans l'acétate d'éthyle (50 mL) et filtré pour éliminer les sels de fer qui sont ensuite lavés à l'acétate d'éthyle. Les phases organiques sont alors combinées et concentrées sous pression réduite pour fournir une huile incolore (4.23 g, 80%, mélange de lactone III, de son formate et de son acétate). Cette huile est ensuite reprise dans l'éthanol (40 mL) puis est ajouté l'IRP-69 (H+) (600 mg) et le mélange est porté à reflux pendant 1 heure avant d'être refroidi à l'ambiante. L'éthanol est évaporé sous pression réduite et le résidu est redissout dans le dichlorométhane (10 mL) et agité pendant 16 heures. Le mélange est alors filtré et concentré sous pression réduite pour donner (S)-4-hydroxyméthylbutyrolactone sous la forme d'une huile incolore (75%).

### Exemple 3 : synthèse de (S)-(5-oxotetrahydrofuran-2-yl)methyl 4-methylbenzenesulfonate de formule (IVa) à partir de 4-hydroxyméthylbutyrolactone de formule (III)

Du chlorure de tosyle (1,55 g, 8,1 mmol) est ajouté en une portion dans une solution constituée par un mélange de 4-hydroxyméthylbutyrolactone (0,9 g, 7,7 mmol) dans du DCM (5 mL)/pyridine (1,5 mL) à température ambiante sous azote. Le mélange réactionnel est agité à température ambiante pendant 4 heures. Le DCM (20 mL) est ajouté et le mélange réactionnel est rincé avec une solution d'HCl 3M, de saumure, séché sur du sulfate de magnésium anhydre, filtré et concentré jusqu'à obtenir un produit sec. Ce produit brut est ensuite trituré avec de l'éther diéthylique (15 mL). Le précipité blanc généré est récupéré par filtration et séché pour obtenir un produit pur de (S)-(5-oxotetrahydrofuran-2-yl)methyl 4-methylbenzenesulfonate de formule (IVa) (1,3 g, 62%) qui est ensuite utilisé en l'état, sans autre purification pour la suite de la synthèse.
¹H NMR (500 MHz): 7.8 ppm (2H, d) ; 7.4 ppm (2H, d) ; 4.7 ppm (1H, m); 4.2 ppm (2H, m); 2.6 ppm (2H, m); 2.5 ppm (3H,s); 2.4 ppm (1H, m); 2.1 ppm (1H, m)

### Exemple 4 : Synthèse de (S)-5-(iodomethyl)dihydrofuran-2(3H)-one (IV-bis-a)

De la triphénylphosphine (524 mg, 2 mmol) est ajoutée à une solution de 4-hydroxymethylbutyrolactone (116 mg, 1 mmol), d'iode (508 mg, 2 mmol) et d'imidazole (136 mg, 2 mmol) dans de l'acétonitrile (10 mL) à 0 °C. Le mélange est ensuite porté à reflux toute une nuit avant d'être ramené à température ambiante puis extrait à l'éther (3 x 20 mL). Les phases organiques sont ensuite rassemblées, lavées à l'eau (20 mL), à la saumure (20 mL) puis séchées sur du sulfate de magnésium anhydre. Après filtration et concentration sous pression réduite, le produit brut est purifié par flash chromatographie (3:1 hexane:acétate d'éthyle) sur gel de silice pour donner, après combinaison et évaporation des fractions pures, le produit de formule (IV-bis-a) sous forme d'une huile incolore (160 mg, 71%).

### Exemple 5 : synthèse du Glycidyl ester de formule (Va) à partir du (S)-(5-oxotetrahydrofuran-2-yl)methyl 4-methylbenzenesulfonate de formule (IVa)

Du methoxide de sodium (0,7g, 13 mmole) est ajouté en une portion à un mélange de (S)-(5-oxotetrahydrofuran-2-yl)methyl 4-methylbenzenesulfonate de formule (IVa) tosylé (3 g, 11 mmol) dans du méthanol (20 mL) à 0 °C sous azote. Le mélange réactionnel est mélangé à température ambiante pendant une heure. De l'acétate d'éthyle (100 mL) est ajouté et le mélange réactionnel est désactivé avec une solution aqueuse saturée en chlorure d'ammonium (50 mL). Les phases sont séparées et la phase aqueuse est à nouveau extraite avec de l'acétate d'éthyle. Les phases organiques combinées sont rincées avec de la saumure puis séchées sur du sulfate de magnésium anhydre, filtrées et concentrées par séchage pour obtenir un époxyde de glycidyl ester (0,9 g, 63%) comme huile. Ce produit brut est utilisé en l'état pour synthétiser le produit d'intérêt suivant.
¹H NMR (500 MHz): 3.7 ppm (3H, s); 3.0 ppm (1H, m); 2.78 ppm (1H, t); 2.52 ppm (1H, dd); 2.48 ppm (2H, t); 2.0 ppm (1H,m); 1.8 ppm (1H, m)

### Exemple 6 : synthèse du Glycidyl ester de formule (Va) à partir du (S)-5-(iodomethyl)dihydrofuran-2(3H)-one (IV-bis-a)

Du methoxide de sodium (0,7g, 13 mmole) est ajouté en une portion à un mélange de (S)-5-(iodomethyl)dihydrofuran-2(3H)-one (IV-bis-a) (2,5 g, 11 mmol) dans du méthanol (20 mL) à 0 °C sous azote. Le mélange réactionnel est mélangé à température ambiante pendant une heure. De l'acétate d'éthyle (100 mL) est ajouté et le mélange réactionnel est désactivé avec une solution aqueuse saturée en chlorure d'ammonium (50 mL). Les phases sont séparées et la phase aqueuse est à nouveau extraite avec de l'acétate d'éthyle. Les phases organiques combinées sont rincées avec de la saumure puis séchées sur du sulfate de magnésium anhydre, filtrées et concentrées par séchage pour obtenir un époxyde de glycidyl ester (0,9 g, 63%) comme huile. Ce produit brut est utilisé en l'état pour synthétiser le produit d'intérêt suivant.
¹H NMR (500 MHz): 3.7 ppm (3H, s); 3.0 ppm (1H, m); 2.78 ppm (1H, t); 2.52 ppm (1H, dd); 2.48 ppm (2H, t); 2.0 ppm (1H,m); 1.8 ppm (1H, m)

### Exemple 7 : synthèse de methyl (S)-4-hydroxyundec-5-ynoate de formule (VIa) à partir de glycidyl ester de formule (Va)

Du n-Buli (2,5 mol/L, dans de l'hexane, 3 mL, 8.3 mmol) a été ajouté dans un mélange de hept-1-yne (750 mg, 7.8 mmol) dans du THF sec (10 mL) à -78 °C sous azote. Le mélange réactionnel est mélangé à la même température pendant 20 min. Du BF₃Et₂O (1 mL, 8.3 mmol) est ensuite ajouté à -78 °C. La réaction est poursuivie à cette même température pendant 20 min avant que de l'époxyde pur de glycidyl ester (1 g, 7.7 mmol) obtenu précemment soit incorporé. La réaction est poursuivie pendant 2 h à -78 °C. Le mélange réactionnel, revenu à -10 °C est alors inactivé avec une solution aqueuse saturée en chlorure d'ammonium (10 mL) et extraite avec de l'acétate d'éthyle (3x30 mL).

Les phases organiques combinées sont rincées à la saumure, séchées sur du sulfate de magnésium anhydre, filtrées et concentrées par séchage. Le produit brut (absorbé sur celite) est purifié par flash chromatographie (5 à 30% d'EtOAc avec du cyclohexane comme éluant) sur gel de silice pour donner, après combinaison et évaporation des fractions pures, le PRODUIT 6 (700 mg, 40%) sous forme d'huile incolore.

### Exemple 8 : synthèse de (S)-5-(oct-2-yn-1-yl)dihydrofuran-2(3H)-one de formule (VIIa) à partir du methyl (S)-4-hydroxyundec-5-ynoate de formule (VIa)

Un mélange de methyl (S)-4-hydroxyundec-5-ynoate de formule (VIa) (600 mg, 2.6 mmol) dans 5 mL d'une solution aqueuse d'acide acétique à 80% est chauffée sous agitation à 50 °C pendant 18 heures. De l'acétate d'éthyle (20 mL) est ajouté et le mélange réactionnel est rincé avec une solution aqueuse saturée en bicarbonate de sodium, de la saumure, puis séché sur du sulfate de magnésium anhydre, filtré puis concentré par séchage. Le produit brut (absorbé sur celite) est purifié par flash chromatographie (5 à 30% d'EtOAc avec du cyclohexane comme éluant) sur gel de silice pour donner, après combinaison et évaporation des fractions pures, une lactone appelée (S)-5-(oct-2-yn-1-yl)dihydrofuran-2(3H)-one de formule (VIIa) (275 mg, 53%) sous forme d'huile incolore.

### Exemple 9 : synthèse de la (Z,S)-dairy-lactone à partir du (S)-5-(oct-2-yn-1-yl)dihydrofuran-2(3H)-one de formule (VIIa)

Un mélange de (S)-5-(oct-2-yn-1-yl)dihydrofuran-2(3H)-one de formule (VIIa) (270 mg, 1.39 mmol) dans 10 mL d'acétate d'éthyle est hydrogéné sous flux de dihydrogène sous agitation avec le catalyseur de Lindlar (50 mg) à température ambiante pendant 6 heures. Le mélange réactionnel est filtré sur un filtre de celite et concentré par séchage pour obtenir la (Z)-dairy-lactone pure (270 mg, 90%) sous forme d'huile incolore. La dairy-lactone obtenue est sous forme Z-S.
¹H NMR (500 MHz): 5.6 ppm (1H, m) ; 5.35 ppm (1H, m) ; 2.55 ppm (3H, m); 2.4 ppm (1H, m); 2.3 ppm (1H, m); 2.05 ppm (2H,m); 1.9 ppm (1H, m); 1.2-1.4 ppm (7H, m), 0.9 ppm (3H, t)

## Revendications

1. Procédé de préparation d'un précurseur d'un unique isomère de dairy-lactone, de formule (VI) ou (VII) où R₁ et R₃ sont des groupements alkyle et où les lignes en pointillée représentent une liaison carbone-carbone simple ou double, comportant une étape de préparation d'un glycidyl-ester de formule (V) sous forme isomérique pure uniquement de conformation R ou S, suivie d'une étape d'ouverture d'époxyde, dans laquelle lesdits précurseurs sont représentés par les formules suivantes :

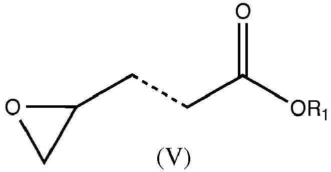

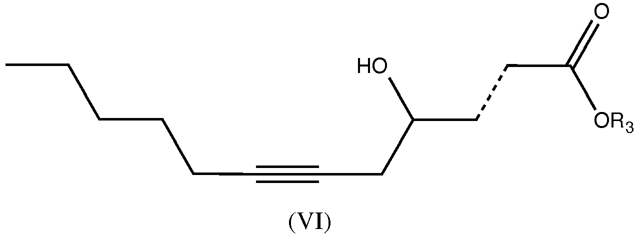

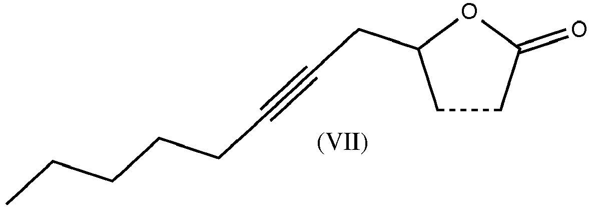

2. Procédé selon la revendication 1 dans lequel le glycidyl-ester est préparé à partir d'une lactone de formule (III) uniquement de conformation R ou S, où la ligne en pointillée représente une liaison carbone-carbone simple ou double, de sorte que ledit glycidyl-ester et ledit précurseur soient sous forme isomérique pure uniquement de forme R ou S, et que la dairy-lactone soit un isomère pur uniquement de forme R ou S correspondante.

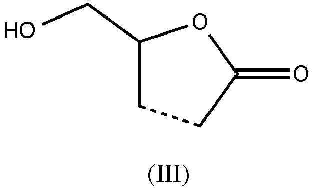

3. Procédé selon l'une au moins des revendications précédentes **caractérisé en ce que** ladite étape de synthèse d'un glycidyl-ester consiste à l'ouverture de la lactone et l'élimination concertée d'un alcool activé d'une molécule de formule (IV) où R₂ est choisi parmi les groupements tosyl ou mésyl et où la ligne en pointillée représente une liaison carbone-carbone simple ou double.

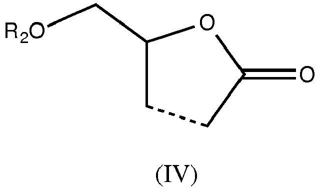

4. Procédé selon l'une au moins des revendications 1 ou 2 **caractérisé en ce que** ladite étape de synthèse d'un glycidyl-ester consiste à l'ouverture de la lactone et l'élimination concertée d'un halogénure d'une molécule de formule (IV-Bis), où X est choisi parmi Br, Cl ou I et où la ligne en pointillée représente une liaison carbone-carbone simple ou double, ladite molécule de formule (IV-Bis) étant obtenue par la substitution par un halogène de l'alcool d'une lactone de formule (III).

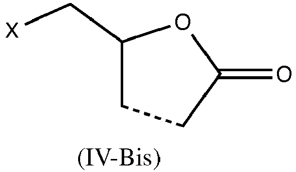

5. Procédé selon l'une des revendications 3 ou 4, **caractérisé en ce que** ladite molécule de formule (IV) est obtenue par activation de l'alcool d'une molécule de formule (III) où la ligne en pointillée représente une liaison carbone-carbone simple ou double.

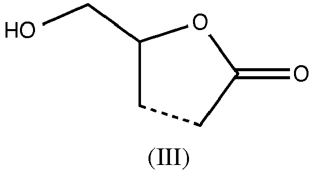

6. Procédé selon la revendication 5 **caractérisé en ce que** ladite molécule de formule (III) dans laquelle la ligne en pointillée représente une liaison carbone-carbone simple est obtenue par hydrogénation d'une lactone α,β-insaturée.

7. Procédé selon la revendication 5 **caractérisé en ce que** ladite molécule de formule (III) est obtenue par oxydation d'une dihydrolévoglucosénone en présence d'un péracide suivie d'une hydrolyse, ladite dihydrolévoglucosénone étant obtenue par hydrogénation d'une lévoglucosénone.

8. Procédé de préparation d'un précurseur de formule (VII) d'un unique isomère de dairy lactone selon l'une au moins des revendications précédentes **caractérisé en ce qu'**il comporte en outre une étape de lactonisation d'une molécule de formule (VI) où R₃ est un groupement alkyle et où la ligne en pointillée représente une liaison carbone-carbone simple ou double pour obtenir une molécule de formule (VII).

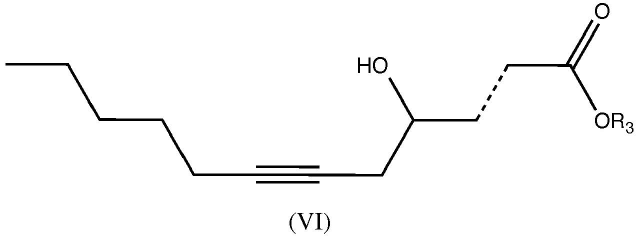

9. Procédé de préparation d'un unique isomère de dairy-lactone comprenant les étapes de :
- de préparation d'un glycidyl-ester de formule (V) sous forme isomérique pure uniquement de conformation R ou S, suivie d'une étape d'ouverture d'époxyde menant au composé (VI)
- de lactonisation d'une molécule de formule (VI) où R₃ est un groupement alkyle et où la ligne en pointillée représente une liaison carbone-carbone simple ou double pour obtenir une molécule de formule (VII)
- syn-hydrogénation de la molécule de formule (VII)

10. Procédé de préparation d'un unique isomère de dairy-lactone selon la revendication 9 **caractérisé en ce que** l'isomère de dairy-lactone obtenu est pur et de forme Z-R, Z-S, E-R ou E-S.

## Patentansprüche

1. Zubereitungsverfahren eines Vorläufers eines einzelnen Dairy-Lacton-Isomers mit der Formel (VI) oder (VII), bei der R₁ und R₃ Alkyl-Anordnungen sind und bei der die gestrichelten Linien eine einfache oder doppelte Kohlenstoff-Kohlenstoff-Verbindung darstellen, umfassend einen Zubereitungsschritt eines Glycidyl-Esters mit der Formel (V) in reiner isomerischer Form nur der Konformation R oder S, gefolgt von einem Epoxid-Öffnungsschritt, bei dem die genannten Vorläufer durch die folgenden Formeln dargestellt sind:

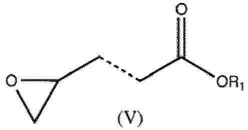

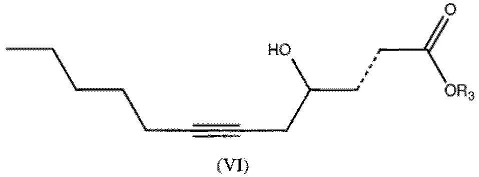

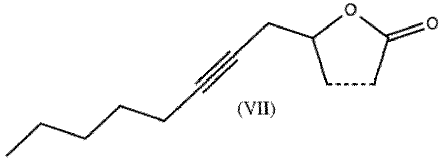

2. Verfahren gemäß Anspruch 1, bei dem der Glycidyl-Ester ausgehend von einem Lacton mit der Formel (III) nur der Konformation R oder S zubereitet ist, bei der die gestrichelte Linie eine einfache oder doppelte Kohlenstoff-Kohlenstoff-Verbindung derart darstellt, dass der genannte Glycidyl-Ester und der genannte Vorläufer in reiner isomerischer Form der Form R oder S vorliegen und dass das Dairy-Lacton ein reines Isomer nur in der entsprechenden Form R oder S vorliegt.

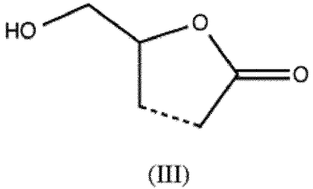

3. Verfahren gemäß wenigstens einem der voranstehenden Ansprüche, **dadurch gekennzeichnet, dass** der genannte Syntheseschritt eines Glycidyl-Esters in der Öffnung des Lactons und der konzertierten Eliminierung eines aktivierten Alkohols eines Moleküls mit der Formel (IV) besteht, bei der R₂ aus den Tosyl- oder Mesyl-Anordnungen ausgewählt ist und bei der die gestrichelte Linie eine einfache oder doppelte Kohlenstoff-Kohlenstoff-Verbindung darstellt.

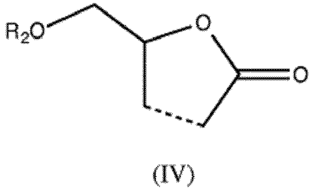

4. Verfahren gemäß wenigstens einem der Ansprüche 1 oder 2, **dadurch gekennzeichnet, dass** der genannte Syntheseschritt eines Glycidyl-Esters in der Öffnung des Lactons und der konzertierten Eliminierung eines Halogenids eines Moleküls mit der Formel (IV-Bis) besteht, bei der X aus Br, Cl oder I ausgewählt ist und bei der die gestrichelte Linie eine einfache oder doppelte Kohlenstoff-Kohlenstoff-Verbindung darstellt, wobei das genannte Molekül mit der Formel (IV-Bis) durch die Substitution durch ein Halogen des Alkohols eines Lactons mit der Formel (III) erhalten ist.

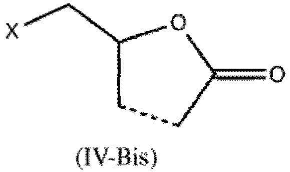

5. Verfahren gemäß einem der Ansprüche 3 oder 4, **dadurch gekennzeichnet, dass** das genannte Molekül mit der Formel (IV) per Aktivierung des Alkohols eines Moleküls mit der Formel (III) erhalten ist, in der die gestrichelte Linie eine einfache oder doppelte Kohlenstoff-Kohlenstoff-Verbindung darstellt.

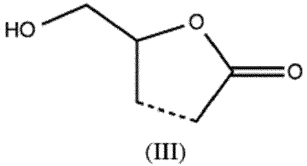

6. Verfahren gemäß Anspruch 5, **dadurch gekennzeichnet, dass** das genannte Molekül mit der Formel (III), bei der die gestrichelte Linie eine einfache Kohlenstoff-Kohlenstoff-Verbindung darstellt, per Hydrierung eines α, β-ungesättigten Lactons erhalten ist.

7. Verfahren gemäß Anspruch 5, **dadurch gekennzeichnet, dass** das genannte Molekül mit der Formel (III) per Oxidation eines Dihydrolevoglucosenons bei Vorhandensein einer Persäure, gefolgt von einer Hydrolyse erhalten ist, wobei das genannte Dihydrolevoglucosenon per Hydrierung eines Levoglucosenons erhalten ist.

8. Zubereitungsverfahren eines Vorläufers mit der Formel (VII) eines einzelnen Dairy-Lacton-Isomers gemäß wenigstens einem der voranstehenden Ansprüche, **dadurch gekennzeichnet, dass** es darüber hinaus einen Lactonisierungsschritt eines Moleküls mit der Formel (VI) umfasst, bei der R₃ eine Alkyl-Anordnung ist und bei der die gestrichelte Linie eine einfache oder doppelte Kohlenstoff-Kohlenstoff-Verbindung darstellt, um ein Molekül mit der Formel (VII) zu erhalten.

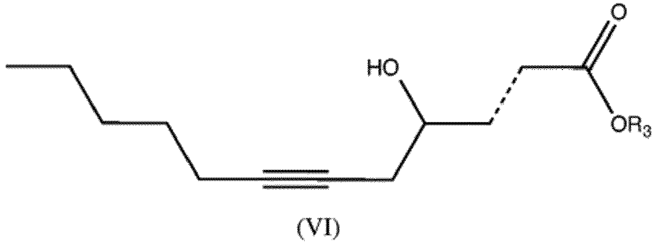

9. Zubereitungsverfahren eines einzelnen Dairy-Lacton-Isomers, umfassend die Schritte:
- der Zubereitung eines Glycidyl-Esters mit der Formel (V) in reiner isomerischer Form nur der Konformation R oder S, gefolgt von einem Epoxid-Öffnungsschritt, der zur Verbindung (VI) führt,
- der Lactonisierung eines Moleküls mit der Formel (VI), bei der R3 eine Alkyl-Anordnung ist und bei der die gestrichelte Linie eine einfache oder doppelte Kohlenstoff-Kohlenstoff-Verbindung darstellt, um ein Molekül mit der Formel (VII) zu erhalten
- syn-Hydrierung des Moleküls mit der Formel (VII).

10. Zubereitungsverfahren eines einzelnen Dairy-Lacton-Isomers gemäß Anspruch 9, **dadurch gekennzeichnet, dass** das erhaltene Dairy-Lacton-Isomer rein ist und die Form Z-R, Z-S, E-R oder E-S aufweist.

## Claims

1. Process for preparing a precursor of a single isomer of dairy-lactone, of formula (VI) or (VII) wherein R₁ and R₃ are alkyl groups and the dotted lines represent a single or double carbon-carbon bond, comprising a preparation step of a glycidylester of formula (V) in a pure isomeric form entirely in R or S conformation, followed by an epoxy opening step, wherein said precursors are represented by the following formulas:

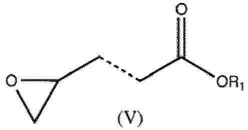

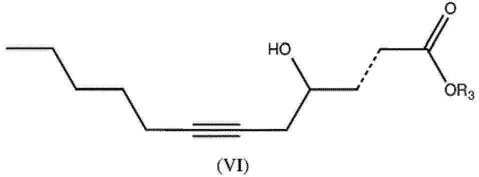

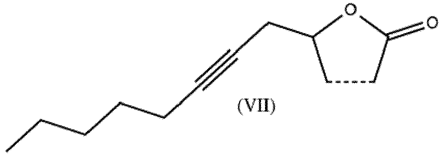

2. Process according to claim 1, in which the glycidylester is prepared from a lactone of formula (III) entirely in R or S conformation, wherein the dotted line represents a single or double carbon-carbon bond, so that said glycidylester and said precursor are in pure entirely R or S isomeric form and that the dairy-lactone is a corresponding pure isomer entirely in R or S form.

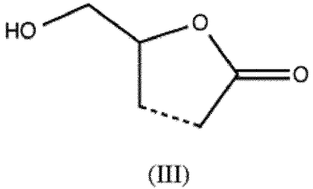

3. Process according to any of the preceding claims, **characterised in that** said step of glycidylester synthesis consists in the opening of the lactone and the concerted removal of an activated alcohol from a molecule of formula (IV), wherein R₂ is selected among mesyl and tosyl groups and the dotted line represents a single or double carbon-carbon bond.

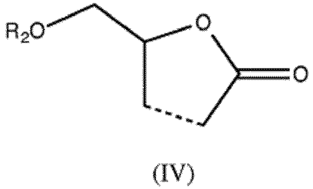

4. The process according to any one of claims 1 and 2, **characterised in that** said glycidylester synthesis step consists in the opening of the lactone and the concerted removal of a halide of a molecule of formula (IV-bis), wherein X is selected among Br, Cl, or I and the dotted line represents a single or double carbon-carbon bond, said molecule of formula (IV-bis) being obtained by the substitution of a halogen in the alcohol of a lactone of formula (III).

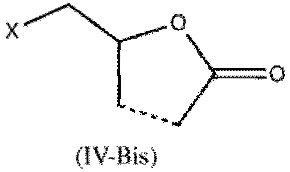

5. Process according to any of claims 3 or 4, **characterised in that** the molecule of formula (IV) is obtained by the activation of the alcohol group of a molecule of formula (III), wherein the dotted line represents a single or double carbon-carbon bond.

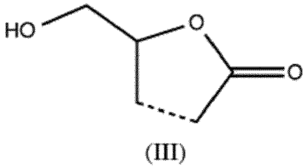

6. Process according to claim 5 **characterised in that** said molecule of formula (III) wherein the dotted line represents a single carbon-carbon bond is obtained by hydrogenation of an α,β-unsaturated lactone.

7. Process according to claim 5 **characterised in that** said molecule of formula (III) is obtained by oxidation of a dihydrolevoglucosenone in the presence of a peracid followed by hydrolysis, said dihydrolevoglucosenone being obtained by hydrogenation of a levoglucosenone.

8. Process for preparing a precursor of formula (VII) of a single isomer of dairy lactone according to any of the previous claims, **characterised in that** it further comprises a step of lactonization of a molecule of formula (VI), wherein R₃ is an alkyl group and the dotted line represents a single or double carbon-carbon bond, to obtain a molecule of formula (VII).

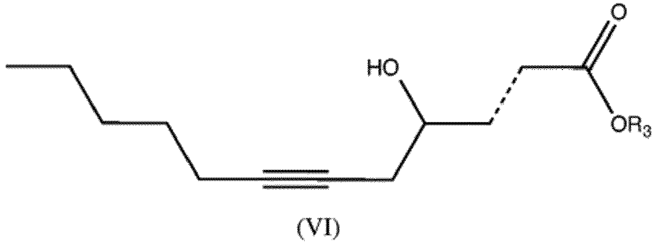

9. Process for preparing a single isomer of dairy-lactone comprising the steps of:
- preparing a glycidylester of formula (V) in an isomeric pure form entirely in R or S conformation, followed by a step of epoxy opening leading to compound (VI)
- lactonization of a molecule of formula (VI) wherein R₃ is an alkyl group and the dotted line represents a single or double carbon-carbon bond, to obtain a molecule of formula (VII)
- syn-hydrogenation of the molecule of formula (VII).

10. Process for preparing a single isomer of dairy-lactone according to claim 9, **characterised in that** the dairy-lactone isomer obtained is pure and in the Z-R, Z-S, E-R or E-S form.
